# EUROPEAN PATENT SPECIFICATION

(11) **EP 0 375 099 B1**
(45) Date of publication and mention of the grant of the patent: **29.09.1993**
(21) Application number: 89305655.6
(22) Date of filing: 05.06.1989
(51) Int. Cl.: C08J 3/14, C12P 19/06, B01F 7/20

(54) **Method and apparatus for purifying and separating a high polymer from a solution thereof**
Verfahren und Vorrichtung zum Reinigen und Abtrennen eines Polymers aus seiner Lösung
Procédé et dispositif pour purifier et sépare un polymère d'une solution

(30) Priority: 22.12.1988 JP 325583/88; 01.04.1989 JP 82248/89
(43) Date of publication of application: 27.06.1990
(73) Proprietor: DAI-ICHI KOGYO SEIYAKU CO., LTD., Shimogyo-ku Kyoto (JP)
(72) Inventor: Sasaki, Masao, Nagaokakyo-shi Kyoto (JP)
(74) Representative: Seaborn, George Stephen

(56) References cited:
- FR-A- 2 228 106
- GB-A- 2 077 125
- US-A- 4 522 505

## Description

### Background of the Invention

The present invention relates to a method and an apparatus for continuously purifying and separating a high polymer from a solution thereof.

It has been known to purify and separate a high polymer by mixing a solution comprising the high polymer with a solvent which is miscible with a solvent comprised in the solution but which does not dissolve the high polymer to precipite the high polymer. This type of purifying and separating method, however, has not been industrially used so widely because it requires much solvent and has low efficiency.

For example, it has been known that xanthan gum, which is a water-soluble natural high polymer, is purified and separated by mixing a xanthan gum culture solution with an organic solvent (isopropyl alcohol) to precipitate the xanthan gum. However, this method has drawbacks that it requires much organic solvent, that the cost for recovering organic solvent is very high and that even if much organic solvent is used, the conventional agitating device causes the precipitated xanthan gum to assume a flocculent form, tending to entwine the stirrer, resulting in very low efficiency, a fact which accounts for the reason why the method has not been used on an industrial scale.

Thus, as in Japanese Patent Publication No.11596 of 1986, various methods including concentration, spray drying, desalting and the like of xanthan gum culture solution are investigated, but all of them have problems from the standpoint of operation and economy, failing to produce xanthan gum of high quality inexpenssively and efficiently.

An object of the present invention is to provide a method and an apparatus for economically and efficiently purifying and separating a high polymer from a solution thereof by mixing the solution with a solvent which is miscible with a solvent comprised in the solution but which does not dissolve the high polymer to precipite the high polymer.

Further, an another object of the present invention is to provide a method and an apparatus useful for purifying and separating xanthan gum by mixing a culture solution thereof directly with an organic solvent (isopropyl alcohol).

### Summary of the Invention

According to the invention, there is provided a method of purifying and separating a high polymer from a high polymer solution comprising the high polymer and a first solvent, the method comprising mixing the polymer solution with a second solvent which does not dissolve the high polymer but which is miscible with the first solvent to precipitate the high polymer, characterized in that the high polymer solution and the second solvent are continuously fed into an agitating and mixing tank and agitated and mixed therein, the high polymer solution being fed through an inner tube and the second solvent being fed, externally of the inner tube, through an outer tube surrounding the inner tube, the high polymer solution and the second solvent being agitated and mixed by upper and lower rotating agitating means, the lower agitating means being downwardly inclined and the inner tube opening into the tank between the upper and lower agitating means. The high polymer is precipitated and simultaneously crushed into small masses to separate the high polymer from the mixed solvents.

According to the invention, there is also provided an apparatus for purifying and separating a high polymer from a high polymer solution comprising the polymer, characterized in that the apparatus comprises an agitating and mixing tank, a rotatable shaft adapted to be driven by a motor being centrally disposed in the tank, the rotating shaft having upper and lower agitating means, an inner tube for supply of the polymer solution opening into the tank between the upper and lower agitating means, an outer tube for supply of a second solvent, externally of the inner tube, surrounding the inner tube and opening into the tank, the lower agitating means being downwardly inclined, and a discharge port for discharge of the mixed solution and second solvent supplied to the tank above the upper agitating means, the lower agitating means being downwardly inclined at an angle of 40 to 80° with respect to the axis of the rotatable shaft.

### Brief Description of the Drawings

Fig. 1 is a principal sectional view showing an example of the apparatus of the invention;
Fig. 2 is a plan view of a lower agitating means used in an example of the invention;
Fig. 3 is a sectional view taken along the line A-A' in Fig. 2;
Fig. 4 to Fig. 6 are respectively a partially sectional view showing a different example of the apparatus of the invention;
Fig. 7 is a plan view of an agitating means used in an example of the invention; and
Fig. 8 is a sectional view taken along the line B-B' in Fig. 7.

### Detailed Description of the Invention

The method of the invention can be efficiently embodied in an apparatus as shown in the accompanying drawings. In an example, a feed tube (8) consisting of a double tube is attached to the bottom region of the lateral wall of an agitating and mixing tank (1) centrally having a rotating shaft (6) which has two agitating means (4a) and (4b) attached thereto one above the other and is adapted to be drived by a motor, in such a manner that the inner tube (2) of the feed tube (8) is used for feeding the high polymer solution (A) and the outer tube (3) is used for feeding the solvent (D) not dissolving the high polymer (B) and the port (2a) of the inner tube for feeding the high polymer solution (A) is positioned between the two agitating means (4a) and (4b), while, in the upper region of the lateral wall of the agitating and mixing tank (1), a mixed liquid discharging port (5) is formed.

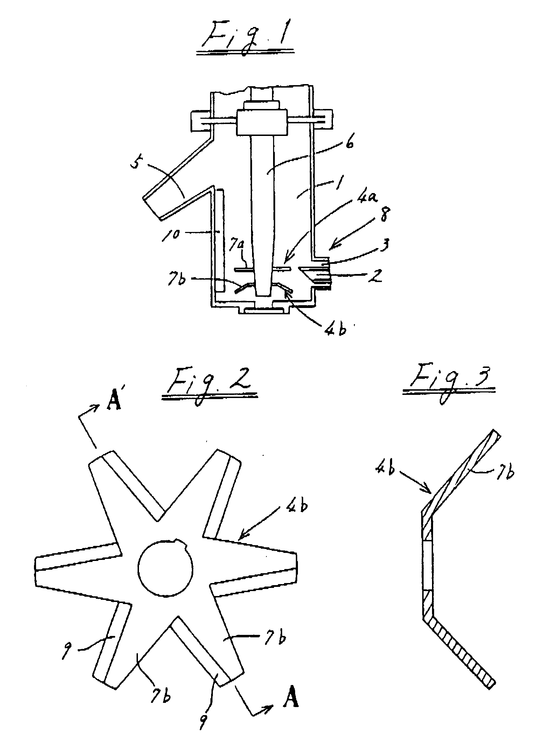

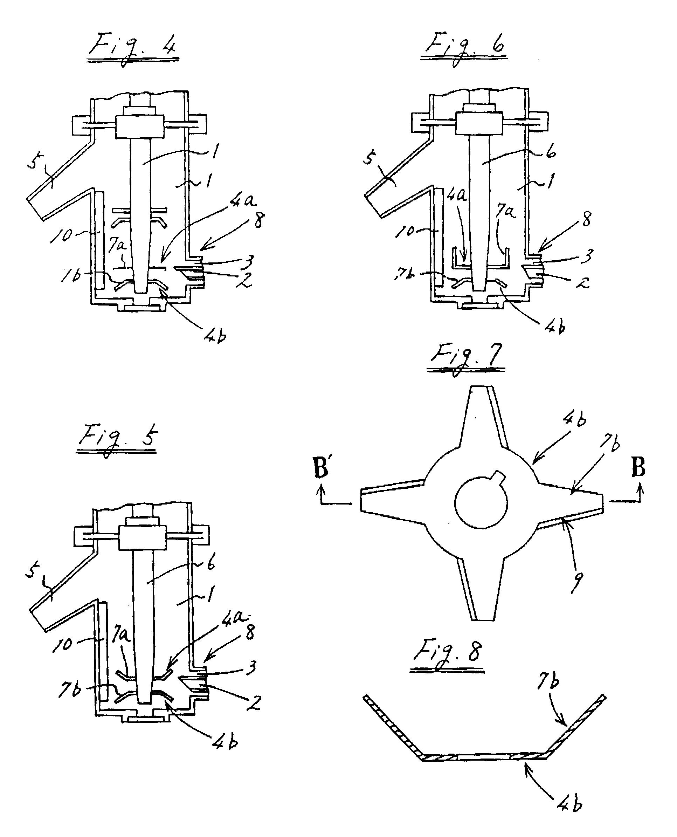

Further, the vanes (7b) of the lower agitating means (4b) are formed downwardly inclined so that they form an angle of 40-80° with respect to the axis of the rotating shaft (6). The vanes (7a) of the upper agitating means (4a) are generally attached to the rotating shaft (6) substantially perpendicularly to its axis (see Fig.1 and Fig.4) but may be formed upwardly inclined (Fig.5 and Fig. 6).

In this way, in the invention, since the feed tube (2) for the high polymer solution (A) and the feed tube (3) for the solvent for precipitating the high polymer (B), are constructed in a double tube, the high polymer solution (A) fed from the feed tube (2) is instantly mixed with the solvent (D) fed from the feed tube (3), whereby the high polymer (B) can be efficiently precipitated.

Further, in the present invention, the two agitating means (4a) and (4b) on the rotating shaft (6) are located in front of the port (2a) of the high polymer solution feed tube (2), so that the high polymer solution (A) fed from the high polymer solution feed tube (2) is drawn into the space between the two agitating means (4a) and (4b) while it is mixed with the solvent (D) for precipitating the high polymer, the solvent (D) being fed from therearound. The high polymer (B) precipitated from the high polymer solution (A) by the mixing with the solvent (D) is obstructed by the upper agitating means (4a) and as soon as it moves downward it is crushed by the lower agitating means (4b). Since the vanes (7b) of the lower agitating means (4b) is downwardly inclined, the high polymer is efficiently precipitated and crushed without soaring.

Thereafter, the high polymer (B) pushed up by the high polymer solution (A) and solvent (D) being continuously fed is finely crushed by the vanes (7a) of the upper agitating means (4a) and concurrently therewith the remainder of the high polymer solvent (C) contained in the high polymer (B) dissolves in and mixed with the solvent (D) introduced from the solvent feed tube (3) and the high polymer (B) becomes purifiable and separable efficiently as by centrifugal separation and is discharged together with the mixture of the solvents (C) and (D) through the mixed liquid discharge port (5) located in the upper region.

In addition, in the apparatus of the invention, the vanes (7a) and (7b) of the agitating means (4a) and (4b) are preferably formed with cutting edges (9) for cutting the high polymer. Further, the rotating shaft (6) may have one or more additional agitating means besides the agitating means (4a) and (4b) (see Fig.4), and the wall surface of the agitating and mixing tank (1) may be provided with a baffle plate.

Further, the vanes (7a) and (7b) of the agitating means (4a) and (4b), as shown in Fig. 7, may have a sharp edge on the front, such as a straight cutting edge, a saw-tooth edge or the like. The number of the vanes (7a) and (7b) attached to an agitating means may be either two or more.

The apparatus of the invention is particularly useful for purifying a high polymer and separating it from an aqueous solution of a water-soluble high polymer by using a water-miscible organic solvent and makes it possible to purify xanthan gum and separate it from a xanthan gum culture solution on an industrial scale.

### Detailed Description of the Preferred Embodiments

The following example serves to illustrate the invention in more detail although the invention is not limited to the example.

### Example 1

A paste (solid content: 6-12%) of a xanthan gum culture solution was continuously fed at a rate of 120 kg/hr by a constant volume pump from the inner tube (2) of the feed tube (8) and concurrently therewith an 80% aqueous solution of isopropyl alcohol was fed at a rate of 240-360 kg/hr by a constant volume pump from the outer tube (3) of the feed tube (8), while the agitating means (4a) and (4b) were rotated at 1500-2000 rpm.

As a result, a uniform slurry liquid with no flocculent sediment was obtained, and xanthan gum of high purity was separated by simple centrifugal separation or filtration.

The lower agitating means (4b) used in this example has six vanes (7b) attached thereto downwardly inclined at an angle of 50° with respect to the axis of the rotating shaft (6) and the front end of each vane (7b) is formed with a cutting edge (9) at an angle of 30° (see Fig.2). In addition, the upper agitating means (4a) also has six vanes (7a), each of which is formed at its front end with a cutting edge (9) at an angle 30° , and the vanes (7a) are attached to the rotating shaft (6) substantially perpendicularly to its axis.

### Comparative Example

A xanthan gum culture solution and 80% isopropyl alcohol, in a ratio of 1 : 3-5, were simultaneously fed to a container having an ordinary agitator, while rotating the agitating means at 1000-1500 rpm. It was found that flocculent masses entwined the agitating means, making it difficult to take out the product.

### Effects of the Invention

In the method and apparatus of the present invention, the high polymer solution (A) is introduced into the agitating and mixing tank in such a manner that it is surrounded by the precipitating solvent (D) for the high polymer (B) contained in the high polymer solution (A), and they are instantly mixed in the space between the two agitating means. As soon as the high polymer (B) is precipitated, it is crushed by the lower agitating means (4b) and the solvent (C) contained in the high polymer (B) mixes with the high polymer precipitating solvent (D) to quickly separate from the high polymer (B); thus, efficient purification and separation of the high polymer (B) is possible.

Therefore, the amount of use of the solvent (D) for purifying the high polymer (B) can be greatly reduced and formation of flocculent masses during purification process can be prevented; thus, it is possible, for example, to effect purification and separation of xanthan gum from a xanthan gum culture solution on an industrial scale.

## Claims

1. A method of purifying and separating a high polymer (B) from a polymer solution (A) comprising the polymer (B) and a first solvent (C), the method comprising mixing the high polymer solution (A) with a second solvent (D) which does not dissolve the high polymer (B) but which is miscible with the first solvent (C) to precipitate the high polymer (B), characterized in that the polymer solution (A) and the second solvent (D) are continuously fed into an agitating and mixing tank (1) and agitated and mixed therein, the high polymer solution (A) being fed through an inner tube (2) and the second solvent (D) being fed, externally of the inner tube, through an outer tube (3) surrounding the inner tube, the high polymer solution and the second solvent being agitated and mixed by upper and lower rotating agitating means (4a and 4b), the lower agitating means (4b) being downwardly inclined, and the inner tube opening into the tank between the upper and lower agitating means (4a, 4b).

2. An apparatus for purifying and separating a high polymer (B) from a high polymer solution (A) comprising the high polymer (B), characterized in that the apparatus comprises an agitating and mixing tank (1), a rotatable shaft (6) adapted to be driven by a motor being centrally disposed in the tank, the rotating shaft having upper and lower agitating means (4a, 4b), an inner tube (2) for supply of the polymer solution (A) opening into the tank between the upper and lower agitating means (4a, 4b), an outer tube (3) for supply of a second solvent (D), externally of the inner tube, surrounding the inner tube and opening into the tank, the lower agitating means being downwardly inclined, and a discharge port (5) for discharge of the mixed solution and second solvent supplied to the tank above the upper agitating means (4a), the lower agitating means being downwardly inclined at an angle of 40 to 80° with respect to the axis of the rotatable shaft.

3. An apparatus as defined in Claim 2, wherein the upper agitating means (4a) has a vane (7a) substantially perpendicular to the rotatable shaft (6).

4. All apparatus as defined in Claim 2, wherein the upper agitating means (4a) has a vane (7a) which is upwardly inclined.

5. All apparatus as defined in Claim 2, wherein agitating means, additional to the agitating means (4a) and (4b), are attached to the rotatable shaft (6).

6. All apparatus as defined in Claim 2, wherein the agitating means (4a, 4b) have sharp front edges.

7. All apparatus as defined in any of claims 2 to 6, wherein the inner tube (2) and the outer tube (3) are mutually coaxial.

## Patentansprüche

1. Verfahren zur Reinigung und Trennung eines Hochpolymers (B) von einer Polymerlösung (A), die das Polymer (B) und ein erstes Lösungsmittel (C) umfaßt, wobei das Verfahren das Mischen der Hochpolymerlösung (A) mit einem das Hochpolymer (B) nicht lösenden zweiten Lösungsmittel (D) umfaßt, das aber mit dem ersten Lösungsmittel (C) zum Fällen des Hochpolymers (B) mischbar ist, dadurch gekennzeichnet, daß die Polymerlösung (A) und das zweite Lösungsmittel (D) kontinuierlich in einen Rühr- und Mischtank (1) eingespeist und darin gerührt und gemischt werden, wobei die Hochpolymerlösung (A) durch ein inneres Rohr (2) geführt und das zweite Lösungsmittel (D) außerhalb des inneren Rohrs durch ein das innere Rohr umgebendes äußeres Rohr (3) geführt wird, wobei die Hochpolymerlösung und das zweite Lösungsmittel durch obere und untere rotierende Rührmittel (4a und 4b) gerührt und gemischt werden, wobei das untere Rührmittel (4b) nach unten geneigt ist und das innere Rohr zwischen den oberen und unteren Rührmitteln (4a, 4b) in den Tank einmündet.

2. Vorrichtung zur Reinigung und Trennung eines Hochpolymers (B) von einer Hochpolymerlösung (A), die das Hochpolymer (B) umfaßt, dadurch gekennzeichnet, daß die Vorrichtung folgendes umfaßt: Einen Rühr- und Mischtank (1); eine drehbare Welle (6), die dergestalt angepaßt ist, daß sie durch einen in der Mitte des Tanks vorgesehenen Motor getrieben werden kann, wobei die sich drehende Welle obere und untere Rührmittel (4a, 4b) besitzt; ein inneres Rohr (2) zur Zuführung der Polymerlösung (A), das zwischen den oberen und unteren Rührmitteln (4a, 4b) in den Tank einmündet; ein äußeres Rohr (3) zur Zuführung eines zweiten Lösungsmittels (D) außerhalb des inneren Rohrs, welches das innere Rohr umgibt und in den Tank einmündet, wobei das untere Rührmittel nach unten geneigt ist; und eine Auslaßöffnung (5) zum Auslaß über dem oberen Rührmittel (4a) der gemischten Lösung und des zweiten Lösungsmittels, die an den Tank zugeführt werden, wobei das untere Rührmittel in einem Winkel von 40 bis 80° in bezug auf die Achse der drehbaren Welle nach unten geneigt ist.

3. Vorrichtung nach Anspruch 2, worin das obere Rührmittel (4a) eine Schaufel (7a) besitzt, die sich im wesentlichen senkrecht zur drehbaren Welle (6) befindet.

4. Vorrichtung nach Anspruch 2, worin das obere Rührmittel (4a) eine nach oben geneigte Schaufel (7a) besitzt.

5. Vorrichtung nach Anspruch 2, worin Rührmittel zusätzlich zu den Rührmitteln (4a) und (4b) an der drehbaren Welle (6) befestigt sind.

6. Vorrichtung nach Anspruch 2, worin die Rührmittel (4a, 4b) scharfe Vorderkanten haben.

7. Vorrichtung nach einem der Ansprüche 2 bis 6, worin das innere Rohr (2) und das äußere Rohr (3) koaxial miteinander sind.

## Revendications

1. Méthode pour purifier et séparer un haut polymère (B) d'une solution polymérique (A) contenant le polymère (B) et un premier solvant (C), où la méthode comprend le mélange de la solution (A) de haut polymère avec un second solvant (D) qui ne dissout pas le haut polymère (B) mais qui est miscible avec le premier solvant (C) de sorte à précipiter le haut polymère (B), caractérisée en ce que la solution polymérique (A) et le second solvant (D) sont introduits sans interruption dans une cuve de mélange et d'agitation (1) et ensuite agités et mélangés dans celle-ci, la solution (A) de haut polymère étant introduite par un tuyau interne (2) et le second solvant (D) étant introduit, au dehors du tuyau interne, par un tuyau externe (3) qui entoure le tuyau interne, la solution de haut polymère et le second solvant étant agités et mélangés au moyen d'agitateurs rotatifs supérieur et inférieur (4a et 4b), l'agitateur inférieur (4b) étant incliné vers le bas, et le tuyau interne débouchant dans la cuve entre les agitateurs supérieur et inférieur (4a, 4b).

2. Appareil pour purifier et séparer un haut polymère (B) d'une solution (A) de haut polymère contenant le haut polymère (B), caractérisé en ce que l'appareil comporte une cuve de mélange et d'agitation (1), un arbre capable de rotation (6) actionné par un moteur étant disposé au centre de la cuve, l'arbre rotatif étant muni d'agitateurs (4a, 4b) supérieur et inférieur, un tuyau interne (2) pour apporter la solution polymérique (A) débouchant dans la cuve entre les agitateurs supérieur et inférieur (4a, 4b), un tuyau externe (3) pour apporter un second solvant (D), en dehors du tuyau interne, entourant le tuyau interne et débouchant dans la cuve, l'agitateur inférieur étant incliné vers le bas, et un orifice de décharge (5) pour décharger la solution et le second solvant en état de mélange apportés à la cuve, au-dessus de l'agitateur supérieur (4a), l'agitateur inférieur étant incliné vers le bas sous un angle de 40° à 80° par rapport à l'axe de l'arbre capable de rotation.

3. Appareil selon la revendication 2, où l'agitateur supérieur (4a) comporte une pale (7a) qui est sensiblement perpendiculaire à l'arbre capable de rotation (6).

4. Appareil selon la revendication 2, où l'agitateur supérieur (4a) comporte une pale (7a) qui est inclinée vers le haut.

5. Appareil selon la revendication 2, où des agitateurs, en supplément des agitateurs (4a) et (4b), sont fixés à l'arbre rotatif (6).

6. Appareil selon la revendication 2, où les agitateurs (4a, 4b) présentent un bord avant tranchant.

7. Appareil selon l'une quelconque des revendications 2 à 6, où le tuyau interne (2) et le tuyau externe (3) sont coaxiaux l'un à l'autre.
